# EUROPÄISCHE PATENTSCHRIFT

(11) **EP 1 476 470 B1**
(45) Veröffentlichungstag und Bekanntmachung des Hinweises auf die Patenterteilung: **22.08.2007**
(21) Anmeldenummer: 03708114.8
(22) Anmeldetag: 20.02.2003
(51) Int. Cl.: C08B 31/00, A61K 31/718, A61K 47/36

(54) **ST RKEDERIVATE, ST RKE−WIRKSTOFF−KONJ UGATE, VERFAHREN ZU IHRER HERSTELLUNG UND IHRE VERWENDUNG ALS ARZNEIMITTEL**
STARCH DERIVATIVES, STARCH ACTIVE SUBSTANCE CONJUGATES, METHOD FOR THE PRODUCTION THEREOF AND THEIR USE AS MEDICAMENTS
DERIVES D'AMIDON, CONJUGUES AMIDON-SUBSTANCES ACTIVES, PROCEDE DE PRODUCTION DE CES CONJUGUES ET LEUR UTILISATION COMME MEDICAMENTS

(30) Priorität: 20.02.2002 DE 10207072
(43) Veröffentlichungstag der Anmeldung: 17.11.2004
(73) Patentinhaber: Fresenius Kabi Deutschland GmbH, 61352 Bad Homburg v.d.H. (DE)
(72) Erfinder: SOMMERMEYER, Klaus, 61191 Rosbach v.d.H. (DE); ZANDER, Nobert, 38527 Meine (DE); FRANK, Ronald, 38527 Meine-Grassel (DE); CONRADT, Harald, 38100 Braunschweig (DE)
(74) Vertreter: Luderschmidt, Schüler & Partner
(86) Internationale Anmeldenummer: PCT/EP2003/001716
(87) Internationale Veröffentlichungsnummer: WO 2003/070772

(56) Entgegenhaltungen:
- DE-A- 10 135 694
- DE-A- 19 628 705
- FR-A- 2 600 897

## Beschreibung

Die vorliegende Erfindung betrifft Stärkederivate, Konjugate aus solchen Stärkederivaten mit Wirkstoffen und ein Verfahren zu ihrer Herstellung. Ferner betrifft die Erfindung die Verwendung der Stärke-Wirkstoff-Konjugate als Arzneimittel.

Die Konjugation von pharmazeutischen Wirkstoffen wie therapeutischen Proteinen, Antibiotika, Nukleinsäuren, Cytokinen oder Hormonen mit Polyethylenglycol-Derivaten ("Pegylierung") ist eine weit verbreitete Methode (Francis G. E. et al., Polyethylene glycol modification in tumor tageting and cytokine therapy., J. Drug Targeting (1995), 3: 321-340). Damit werden beispielsweise an sich wasserunlösliche Wirkstoffe in lösliche Derivate umgewandelt, die dann in die Blutbahn applizierbar sind.

Weiterhin ist es möglich, durch die Kopplung von Polyethylenglycol-Derivaten das Molekulargewicht von Wirkstoffen so zu erhöhen, dass die Filtration über die Niere nicht mehr möglich ist, d.h. dass die sogenannte Nierenschwelle überschritten wird und so die Plasma-Halbwertszeiten solcher Derivate erheblich im Vergleich zu den unkonjugierten Wirkstoffen verlängert werden. Darüber hinaus lassen sich durch die Kopplung mit Polyethylenglycol-Derivaten die Antigenität von z.B. Proteinen nicht menschlichen Ursprungs reduzieren, die ansonsten zu immunologischen Nebenwirkungen bei der Applikation führen würden.

Polyethylenglycol (PEG) hat jedoch den Nachteil, dass es ein nicht metabolisierbares Molekül ist und mit diesem derivatisierte Proteine auch zur Vakuolisierung der Niere führen können. Daher ist es von besonderem Interesse, Derivatisierungen von Wirkstoffen mit metabolisierbaren Polymeren durchzuführen, deren Abbau im Körper vorzugsweise steuerbar ist. Ein geeignetes Molekül hierfür ist Hydroxyethylstärke (HES), welche als Plasmaexpander in verschiedenen molekularen Spezifikationen seit langem breit eingesetzt wird (DE 196 28 705 A1).

HES weist auch bei Applikation in sehr hohen Dosen nur sehr selten und in sehr geringem Umfang Nebenwirkungen auf, verglichen mit anderen Plasmaexpandern, wie z.B. Gelatine-Derivaten oder Dextranen, oder auch Humanalbumin.

Ein weithin ungelöstes Problem bei der Derivatisierung von Wirkstoffen ist jedoch die selektive Anbindung des Wirkstoffs an den Träger. Bei Proteinen ist es beispielsweise wünschenswert, die Kopplung an einen Träger in ausreichender Entfernung vom reaktiven Zentrum oder vom Rezeptor durchzuführen. Ansonsten kann die Aktivität herabgesetzt oder zerstört werden.

De 196 28 705 A1 beschreibt ein Verfahren zur Anbindung von Hämoglobin an Hydroxyethylstärke. Allerdings findet die Anbindung relativ unselektiv über die zahlreichen freien Aminogruppen des Hämoglobins statt.

Diese bilden durch Reaktion mit den in oxidierter Form vorliegenden reduzierenden Endgruppen der Hydroxyethylstärke Amidbindungen aus.

Ferner beschreibt die FR 2600897 ein Verfahren zur Kopplung von Trypsin mit Stärkederivaten, bei dem man die Hydroxylgruppen an den C-Atomen 2 und 3 der Glucopyranose-Fragmente der Stärkederivate mit einem Metaperiodat oxidiert, die oxidierten Funktionen mit einem Alkylendiamin aminiert, die aminierten Gruppen mit Glutaraldehyd aktiviert und das Stärkederivat über die aktivierten Funktionen mit Trypsin koppelt.

In Anbetracht des diskutierten Standes der Technik lag der Erfindung die Aufgabe zugrunde, Stärkederivate zur Verfügung zu stellen, die möglichst selektiv an einen Wirkstoff binden.

Ferner sollte ein solches Stärkederivat so beschaffen sein, dass eine möglichst quantitative Anbindung eines Wirkstoffes durch kovalente Bindung an dieses Stärkederivat stattfindet.

Der Erfindung lag weiterhin die Aufgabe zugrunde, Stärkederivate zur Verfügung zu stellen, deren Abbauverhalten im Organismus steuerbar ist. Insbesondere sollten die Stärkederivate derart beschaffen sein, dass sie die Nierenschwelle nicht passieren können und eine schnelle Ausscheidung verhindert wird. Im Ergebnis sollten die Stärkederivate eine verlängerte Halbwertzeit im Blutserum aufweisen. Dennoch sollten die Stärkederivate innerhalb einer physiologisch vernünftigen Zeit restlos abbaubar sein.

Ebenso sollte das Löslichkeitsverhalten von Wirkstoffen in wässriger Phase und in organischen Lösungsmitteln durch ihre Anbindung an die benannten Stärkederivate in einem weiten Bereich gesteuert werden können.

Schließlich lag der Erfindung die Aufgabe zugrunde ein möglichst einfaches und kostengünstiges Verfahren zur Herstellung solcher Stärkederivate und ihrer Kopplungsprodukte mit Wirkstoffen zur Verfügung zu stellen.

Gelöst werden diese Aufgaben sowie weitere, die zwar nicht wörtlich genannt werden, sich aber aus den hierin diskutierten Zusammenhängen wie selbstverständlich ableiten lassen oder sich aus diesen zwangsläufig ergeben, mit den in Anspruch 1 beschriebenen Stärkederivaten. Zweckmäßige Abwandlungen dieser erfindungsgemäßen Stärkederivate werden in den auf Anspruch 1 rückbezogenen Unteransprüchen 2-9 unter Schutz gestellt.

In den Ansprüchen 10-25 werden Konjugate solcher Stärkederivate mit Wirkstoffen unter Schutz gestellt.

Hinsichtlich eines Verfahrens zur Herstellung der Stärke-Wirkstoff-Konjugate, bei dem man die genannten Stärkederivate als Zwischenprodukt erhält, liefern die Ansprüche 26-28 eine Lösung der zugrunde liegenden Aufgabe.

Die Ansprüche 29-31 beschreiben Arzneimittel, welche die erfindungsgemäßen Stärke-Wirkstoff-Konjugate umfassen und bevorzugte medizinische Verwendungen dieser Arzneimittel.

Durch die Bereitstellung von Verbindungen der Formel (I) 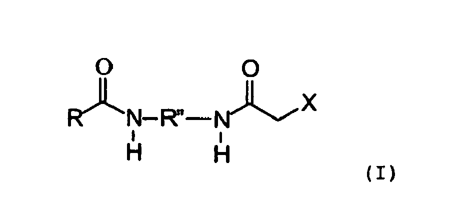 wobei X ein Brom oder Iodatom bedeutet, R" eine geradkettige oder verzweigte Alkyl-, Aryl- oder Aralkylgruppe bedeutet und R-CO- einen oxidierten substituierten oder unsubstituierten Stärkerest bedeutet, der an der reduzierenden Endgruppe zu einer Carbonsäure oxidiert ist, gelingt es Stärkederivate zur Verfügung zu stellen, die äußerst selektiv an die SH-Funktionen von Wirkstoffen binden.

Weiterhin werden durch die erfindungsgemäße Verbindung die folgenden Vorteile erzielt:

Durch die besondere Ausgestaltung der Stärkederivate wird verhindert, dass diese die Nierenschwelle passieren können, wodurch die Halbwertzeit des Wirkstoffs im Blutserum verlängert wird. Die Halbwertszeit beschreibt die Zeit, nach der die Hälfte des eingesetzten Wirkstoffs abgebaut oder ausgeschieden wurde.

Die Verbindungen der Formel (I) sind innerhalb einer physiologisch vernünftigen Zeit restlos abbaubar und weisen auf der anderen Seite dennoch ein steuerbares Eliminationsverhalten auf.

Derivate nach Anspruch 1 lassen sich generell von jeder Stärke herstellen, die eine zu einer Carbonsäure oxidierbare Gruppe aufweist. Vorzugsweise handelt es sich dabei um die reduzierende Endgruppe einer Stärke. Es wurde gefunden, dass die zuvor genannten Eigenschaften der Verbindung (I) besonders gut erzielt werden können, wenn der oxidierte Stärkerest R-CO- ein Hydroxyethylstärkerest ist.

Ausgangsprodukte für die Gewinnung von Hydroxyethylstärke sind Stärken, die einen hohen Gehalt an Amylopektin, der hochverzweigten Komponente von Stärke, aufweisen, insbesondere Kartoffelstärke, Wachsmaisstärke, Sorghumstärke oder wachsartige Reisstärke.

Zur groben Voreinstellung des beabsichtigten Molekulargewichts werden diese Stärken einer hydrolytischen Abbaureaktion unterworfen. Dabei wird das Molekulargewicht von etwa 20.000.000 Dalton auf mehrere Millionen Dalton reduziert.

Bei der anschließenden alkalischen Hydroxyethylierung mit bekannten Hydroxyethylierungsmitteln ist die Einführung einer Hydroxyethylgruppe in Position 2, 3 und 6 der Anhydroglucoseeinheit möglich. Disubstituierte Einheiten, wie 2,3-Dihydroxyethylanhydroglucose, 2,6-Dihydroxyethylanhydroglucose werden dabei mit geringerer Wahrscheinlichkeit bei der Synthese gebildet.

Für die Erfassung der Substitution durch Hydroxyethylgruppen existieren zwei unterschiedlich definierte Substitutionsgrade.

Der Substitutionsgrad MS (molar substitution) ist definiert als die durchschnittliche Anzahl von Hydroxyethylgruppen pro Anhydroglucoseeinheit. Er wird ermittelt aus der Gesamtanzahl der Hydroxyethylgruppen in einer Probe, beispielsweise nach Morgan, durch Ätherspaltung und anschließender quantitativer Bestimmung von Ethyliodid und Ethylen, die hierbei gebildet werden.

Hingegen ist der Substitutionsgrad DS (degree of substitution) definiert als der Anteil der substituierten Anhydroglucoseeinheiten aller Anhydroglucoseeinheiten. Ihn kann man bestimmen aus der gemessenen Menge der unsubstituierten Glucose nach Hydrolyse einer Probe. Aus diesen Definitionen ergibt sich, daß MS > DS. Für den Fall, daß nur Monosubstitution vorliegt, also jede substituierte Anhydroglucoseeinheit nur eine Hydroxyethylgruppe trägt, ist MS = DS.

Ein Hydroxyethylstärkerest innerhalb der Formel (I) der vorliegenden Erfindung weist bevorzugt einen Substitutionsgrad MS von 0,1 bis 0,8 auf. Besonders bevorzugt weist der Hydroxyethylstärkerest einen Substitutionsgrad MS von 0,4 bis 0,7 auf.

Die Reaktivität der einzelnen Hydroxygruppen in der unsubstituierten Anhydroglucoseeinheit gegenüber Hydroxyethylierung ist je nach Reaktionsbedingungen unterschiedlich. Innerhalb gewisser Grenzen ist dadurch das Substitutionsmuster, also die einzelnen, unterschiedlich substituierten Anhydroglucosen, die statistisch auf die einzelnen Polymermoleküle verteilt sind, beeinflußbar. Vorteilhaft werden überwiegend die C₂- und die C₆-Position hydroxyethyliert, wobei die C₆-Position aufgrund ihrer leichteren Zugänglichkeit häufiger substituiert wird.

Vorzugsweise verwendet werden im Rahmen dieser Erfindung überwiegend in C₂-Position substituierte Hydroxyethylstärken (HES), die möglichst homogen substituiert sind. Die Herstellung solcher HES wird in EP 0 402 724 B2 beschrieben. Sie sind innerhalb einer physiologisch vernünftigen Zeit restlos abbaubar und weisen auf der anderen Seite dennoch ein steuerbares Eliminationsverhalten auf. Die überwiegende C₂-Substitution macht die Hydroxyethylstärke relativ schwierig abbaubar für α-Amylase. Es ist von Vorteil, daß möglichst keine innerhalb der Polymermoleküle hintereinander substituieren Anhydroglucoseeinheiten auftreten, um die restlose Abbaubarkeit zu gewährleisten. Weiterhin besitzen solche Hydroxyethylstärken trotz der niedrigen Substitution eine ausreichend hohe Löslichkeit in wässrigem Medium, so daß die Lösungen auch über längere Zeiträume stabil sind und sich keine Agglomerate bzw. Gele bilden.

Bezogen auf die Hydroxyethylgruppen der Anhydroglucoseeinheiten weist ein Hydroxyethylstärkerest innerhalb der Formel (I) der vorliegenden Erfindung bevorzugt ein Verhältnis von C₂:C₆-Substitution im Bereich von 2 bis 12 auf. Besonders bevorzugt beträgt das Verhältnis von C₂:C₆-Substitution 3 bis 11.

Zur Kopplung mit einem Wirkstoff werden Hydroxyethylstärken (HES) bevorzugt an ihrem reduzierenden Ende zur Carbonsäure bzw. zum Lacton oxidiert. De 196 28 705 A1 beschreibt ein Verfahren, in dem HES mit Iod/Kaliumhydroxid am reduzierenden Ende oxidiert wird. Eine anschließende Kopplung an einen Wirkstoff kann über die erhaltene Säurefunktion erfolgen.

Der Rest R-CO- in der erfindungsgemäßen Verbindung der Formel (I) bezeichnet in der bevorzugten Ausgestaltung einen oxidierten Hydroxyethylstärkerest, der an der reduzierenden Endgruppe auf die beschriebene Art und Weise zu einer Carbonsäure oxidiert ist.

Bedingt durch den Einsatz des natürlichen Ausgangsrohstoffes Amylopektin sowie durch das Herstellungsverfahren, bei dem im gewissen Umfang eine Spaltung der Polymerketten notwendig ist, liegt Hydroxyethylstärke nicht als molekulareinheitliche Substanz mit definiertem Molekulargewicht vor, sondern als Gemisch von Molekülen unterschiedlicher Größe, die auch verschieden durch Hydroxyethylgruppen substituiert sind. Die Charakterisierung solcher Gemische bedarf der Zuhilfenahme statistisch gemittelter Größen (vgl. K. Sommermeyer et. al., "Klinisch verwendete Hydroxyethylstärke: Physikalisch-chemische Charakterisierung", Krankenhauspharmazie, 271 (1987)). Zur Kennzeichnung des durchschnittlichen Molekulargewichts dient daher das gemittelte Molekulargewicht Mw. Die allgemeine Definition dieses Mittelwerts lautet:

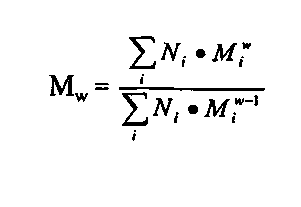

Ein Hydroxyethylstärkerest R-CO- innerhalb der Formel (I) der vorliegenden Erfindung besitzt bevorzugt ein mittleres Molekulargewicht Mw von 2.000 bis 1.000.000 D (bestimmt mit Gelpermeationschromatographie). Noch mehr bevorzugt beträgt das mittlere Molekulargewicht Mw 5.000 bis 500.000 D und am meisten bevorzugt 8.000 bis 250.000 D.

Die Gruppe R" in der Verbindung (I) kann sowohl gesättigte als auch ungesättigte Bindungen enthalten. Ein Alkyl-, Arylrest oder Aralkylrest als R" kann ebenso weitere Substituenten enthalten, wie z.B. Alkyl-, Aryl-, Aralkyl-, Halogen-, Carbonyl-, Acyl-, Carboxyl-, Carboxylester-, Hydroxy, Thiol-, Alkoxy- und/oder Alkylthiosubstituenten. In einer bevorzugten Ausführungsform ist R" eine Gruppe der Formel (CH₂)ₙ, wobei n eine ganze Zahl von 1 bis 10 bedeutet. Besonders bevorzugt ist R" eine Ethylen-, Propylen-, Butylen-, Pentamethylen-, Hexamethylen- oder Oktamethylengruppe.

Die Erfindung betrifft ebenfalls Stärke-Wirkstoff-Konjugate der allgemeinen Formel (II) 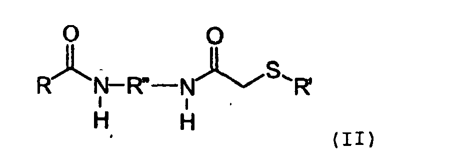 wobei R" eine geradkettige oder verzweigte Alkyl-, Aryl- oder Aralkylgruppe bedeutet, R-CO- einen oxidierten substituierten oder unsubstituierten Stärkerest bedeutet, der an der reduzierenden Endgruppe zu einer Carbonsäure oxidiert ist, und R' der Rest eines Wirkstoffs ist.

Die Stärke-Wirkstoff-Konjugate der Formel (II) sind Kopplungsprodukte aus den zuvor beschriebenen Verbindungen der Formel (I) und einem Wirkstoff, welcher mindestens eine SH-Gruppe enthält. Die Reste R-CO- und R" haben die selben Bedeutungen wie bereits zuvor anhand der Formel (I) erläutert.

Bevorzugte Wirkstoffe R'-SH, die als Rest R'-S- in den Verbindungen der Formel (II) enthalten sind, sind ausgewählt aus einem Peptid, einem Protein, einem Antibiotikum, einer Nukleinsäure, oder einem Hormon. Voraussetzung ist, dass diese Verbindungen mindestens eine SH-Gruppe enthalten.

Es kann sich ebenso um ein Protein oder Petid handeln, dem ein Cysteinrest durch gezielte Mutagenese eingeführt wurde. Sofern in Proteinen oder Peptiden keine SH-Gruppen vorhanden sind, können im Rahmen der vorliegenden Erfindung sogenannte Cysteinmuteine von therapeutischen Proteinen verwendet werden, bei denen durch gentechnisch gezielte Mutagenese ein Austausch bzw. eine Einführung von Cysteinresten gezielt durchgeführt wurde. Ein solcher Austausch ist der Fachwelt bekannt und u.a. beschrieben in: A. Bendele et al., Short Communication: Renal Tubular Vacuolation in Animals Treaded with Polyethylene-Glycol-conjugated Proteins, Toxicological Sciences 42, 152-157 (1998).

Ebenso können SH-Funktionen in Wirkstoffe, die eine primäre Aminogruppe tragen, durch Umsetzung mit 2-Iminothiolan (Trauts-Reagenz) eingeführt werden, bevor man die Wirkstoffe mit Verbindungen der Formel (I) umsetzt. Die Einführung von SH-Funktionen in Wirkstoffe, wie z.B. in Proteine, mittels dieser Methode ist der Fachwelt allgemein bekannt.

Bevorzugte Wirkstoff-Proteine sind therapeutische Antikörper, Antikörper-Fab-Fragmente oder Antikörper-F(ab')₂-Fragmente. Solche Antikörper Fragmente sind wegen ihres relativ kleinen Molekulargewichts leicht nierengängig und können durch die Derivatisierung mit Stärke in ihrer Serum-Halbwertszeit verlängert werden. Ebenso wurde im Rahmen der vorliegenden Erfindung festgestellt, dass der hydrolytische Abbau der Antikörper bzw. der Antikörperfragmente durch Proteasen mit Hilfe der Derivatisierung mit Hydroxyethylstärke verringert werden kann.

In weiteren bevorzugten Ausführungsformen handelt es sich bei dem Wirkstoff um ein Cytokin, insbesondere um ein Interferon α 2a oder ein Interferon α 2b, oder um Erytropoetin.

Im Rahmen der vorliegenden Erfindung wurde festgestellt, dass die Löslichkeit eines Wirstoffs im wäßrigen Medium beeinflusst werden kann, wenn man diesen an eine Verbindung der Formel (I) koppelt und in ein Stärke-Wirkstoff-Konjugat der Formel (II) überführt.

Im Rahmen der Erfindung wurde ebenfalls festgestellt, dass die Löslichkeit eines Proteins oder Enzyms in organischen Lösungsmitteln erhöht werden kann, wenn man das Protein oder Enzym an eine Verbindung der Formel (I) koppelt und in ein Stärke-Wirkstoff-Konjugat der Formel (II) überführt. Bevorzugte aprotische Lösungsmitteln sind Dimethylformamid, Dimethylsulfoxid oder Dimethylacetamid.

Die vorliegende Erfindung betrifft in einem weiteren Aspekt ebenso ein Verfahren zur Herstellung der vorangehend beschriebenen Stärke-Wirkstoff-Konjugate der Formel (II). Als Zwischenprodukt dieses Verfahrens wird das eingangs beschriebene Stärkederivat der Formel (I) erhalten. Das Verfahren ist durch folgende Schritte gekennzeichnet:
a) Zunächst werden die reduzierenden Endgruppen einer substituierten oder unsubstituierten Stärke selektiv zur Carboxyl- oder Lactongruppe oxidiert. Bevorzugt wird Hydroxyethylstärke eingesetzt. Die Oxidation kann beispielsweise mit Iod/Kaliumhydroxid entsprechend DE 196 28 705 A1 erfolgen.
b) Die in Schritt a) erhaltene oxidierte Stärke oder Hydroxyethylstärke wird an ihrer Carboxylgruppe oder Lactongruppe mit einem Diamin

   H₂R-R"-NH₂

   umgesetzt, wobei R" einen Alkyl-, Aryl- oder Aralkylrest bedeutet, welcher verzweigt oder unverzweigt sein kann. Ebenso können die genannte Reste sowohl gesättigte als auch ungesättigte Bindungen enthalten. Ein Alkylrest, Arylrest oder Aralkylrest kann ebenso weitere Substituenten enthalten, wie z.B. Alkyl-, Aryl-, Aralkyl-, Halogen-, Carbonyl-, Acyl-, Carboxyl-, Carboxylester-, Hydroxy, Thiol-, Alkoxy- und/oder Alkylthiosubstituenten.
   Vorzugsweise ist R" ein unverzweigter gesättigter Alkylrest (CH₂)ₙ, wobei n eine ganze Zahl von 2 bis 10 bedeutet. Besonders bevorzugte Verbindungen sind Ethylendiamin, 1,3-Diaminopropan, 1,4-Diaminobutan, 1,5-Diaminopentan, 1,6-Diaminohexan und 1,8-Diaminooktan.
   Durch die Umsetzung der oxidierten substituierten oder unsubstituierten Stärke mit dem zuvor beschriebenen Diamin erhält man eine Verbindung der Formel (III) 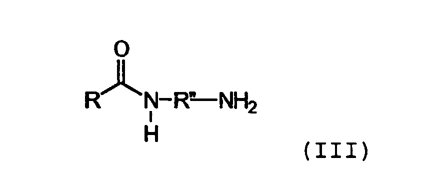 in der R-CO- einen, wie schon eingangs anhand der Formel (I) beschriebenen, oxidierten substituierten oder unsubstituierten Stärkerest darstellt, der an der reduzierenden Endgruppe zu einer Carbonsäure oxidiert ist.
c) Die Verbindung der Formel (III) wird mit einer Halogenessigsäure und 1-Ethyl-3-(3-dimethylaminopropyl) carbodiimid als Aktivator zu einer Verbindung der Formel (I) umgesetzt 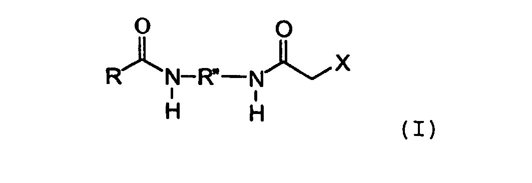 bei der X ein Brom oder Iodatom bedeutet.
d) Schließlich wird die Verbindung der Formel (I) mit einem Wirkstoff mit mindestens einem Thiolrest R'-SH zu einem Stärke-Wirkstoff-Konjugat der allgemeinen Formel (II) umgesetzt. 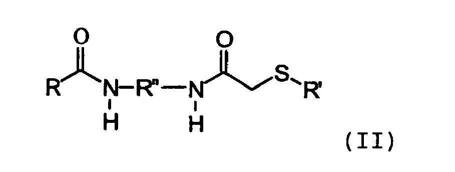 wobei R' einen Wirkstoffrest darstellt.

Es wurde gefunden, dass unter neutralen bis leicht alkalischen Bedingungen die Thiolgruppe eines Wirkstoffs schneller als andere reaktive Gruppen mit Verbindungen der Formel (I) reagiert. Vorzugsweise beträgt der pH-Wert 6,5-8,5. Unter diesen Bedingungen findet eine Deprotonierung der Thiolgruppe zum Thiolation statt, welches besonders reaktiv ist und selektiv mit Verbindungen der Formel (I) reagiert.

Es wurde festgestellt, dass mit dem voranstehend beschriebenen Verfahren die Derivatisierung eines Wirkstoffs durch Kopplung an eine Stärke äußerst selektiv durchgeführt werden kann. Selektiv bedeutet in diesem Fall, dass ein Wirkstoff im wesentlichen nur über seine Thiolgruppen mit Verbindungen der Formel (I) reagiert und dass die Kopplung zum Stärke-Wirkstoff-Konjugat im wesentlichen nur über Thioetherbindungen stattfindet.

Besonders bevorzugt wird das Kopplungsverfahren mit SH-Gruppen enthaltenden Peptiden oder Proteinen durchgeführt. Dabei ist eine Reaktion der Verbindung (I) auch mit SS-Gruppen eines Proteins oder Peptids möglich, nachdem diese in SH-Gruppen überführt wurden.

Die Ausbeuten der Umsetzung von Verbindungen der Formel (I) mit einem SH-Gruppen enthaltenden Peptid oder Protein betragen je nach Molekulargewicht des Proteins oder Peptids und der Anzahl der SH- oder SS-Gruppen zwischen 20% und 90%. Im günstigen Fall ist demzufolge eine weitgehend quantitative Kopplung eines Wirkstoffs an den Stärketräger erreichbar.

Es ist ebenso möglich, ein Zwischenprodukt der Formel (III) in Schritt c) des oben beschriebenen Verfahrens statt mit einer Halogenessigsäure mit anderen gebräuchlichen Vernetzungsmitteln umzusetzen. In diesem Fall reagiert eine funktionelle Gruppe des Vernetzungsmittels mit der primären Aminogruppe der Verbindung (III). Im folgenden Schritt reagiert eine der übrigen funktionellen Gruppen des Vernetzungsmittels mit einer funktionellen Gruppe eines Wirkstoffes, vorzugsweise mit einer SH-Gruppe, wodurch ein Stärke-Wirkstoff-Konjugat gebildet wird. Gebräuchliche Vernetzungsmittel sind z.B. bifunktionelle Vernetzungsmittel mit α-ω-endständigen gleichartigen oder verschiedenen funktionellen Gruppen. Eine Übersicht über solche Vernetzungsmittel ist im Katalog der Firma Perbio (2001/2002) zu finden.

Weiterhin ist es möglich und für den Fachmann selbstverständlich, einen eingangs beschriebenen Stärkerest oder Hydroxyethylstärkerest, der an der reduzierenden Endgruppe zu einer Carbonsäure oxidiert ist, direkt mit einem der oben beschriebenen gebräuchlichen Vernetzungsmittel umzusetzen. In diesem Fall reagiert eine funktionelle Gruppe des Vernetzungsmittels mit der Carboxylgruppe der oxidierten Stärke bzw. Hydroxyethylstärke. Im folgenden Schritt reagiert eine der übrigen funktionellen Gruppen des Vernetzungsmittels mit einer funktionellen Gruppe eines Wirkstoffes, vorzugsweise mit einer SH-Gruppe, wodurch ein Stärke-Wirkstoff-Konjugat gebildet wird.

Gemäß einem Aspekt der vorliegenden Erfindung werden die zuvor beschriebenen Stärkederivate von Wirkstoffen zur Herstellung eines Arzneimittels verwendet. Vorzugsweise handelt es sich dabei um ein Arzneimittel zur Behandlung von Infektionskrankheiten oder hormoneller Störungen. In diesem Zusammenhang kann ein solches Arzneimittel übliche pharmazeutische Hilfsstoffe enthalten.

Nachfolgend wird die Erfindung durch ein Beispiel beschrieben, ohne dass die Erfindung auf dieses beschränkt werden soll.

### Beispiel 1:

10 g analog DE 196 28 705 A1 hergestelltes Konjugat aus oxidierter Hydroxyethylstärke mit einem mittleren Molekulargewichtes Mw von 40.000 und einem Substitutionsgrad MS von 0,2 wurden zusammen mit Ethylendiamin in 50 ml destilliertem Wasser gelöst. 0,2 g Bromessigsäure wurden in 5 ml destilliertem Wasser gelöst, der pH-Wert mit 0,01 normaler Natronlauge auf 4,5 eingestellt und diese Lösung zu der oben beschriebenen aminofunktionalisierten Hydroxyethylärke gegeben. Während des Rührens wurden 0,1 g 1-Ethyl-3-(3-dimethylaminopropyl)carbodiimid zur Reaktionsmischung gegeben, und der pH-Wert für eine Stunde durch Zugabe von 0,01 normaler Salzsäure und anschließend 0,01 normaler Natronlauge bei 4,5 gehalten. Nach weiteren 2 Stunden Reaktionszeit wurde das Reaktionsprodukt ultrafiltriert und danach mit Ethanol gefällt und gewaschen und unter Lichtschutz im Vakuum getrocknet.

## Patentansprüche

1. Stärkederivate der Formel (I) 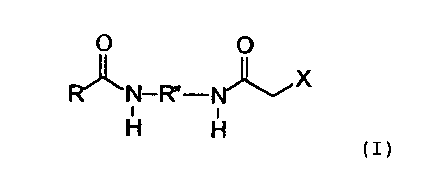 wobei X ein Brom oder Iodatom bedeutet, R" eine geradkettige oder verzweigte Alkyl-, Aryl- oder Aralkylgruppe bedeutet und R-CO- einen oxidierten substituierten oder unsubstituierten Stärkerest bedeutet, der an der reduzierenden Endgruppe zu einer Carbonsäure oxidiert ist.

2. Stärkederivate nach Anspruch 1, wobei R" eine Gruppe der Formel (CH₂)ₙ ist und n eine ganze Zahl von 1 bis 10 bedeutet.

3. Stärkederivate nach Anspruch 1 oder 2, wobei der Rest R-CO- ein zur Carbonsäure oxidierter Hydroxyethylstärkerest ist, der ein Molekulargewicht Mw von 2.000 bis 1.000.000 D aufweist.

4. Stärkederivate nach Anspruch 1 oder 2, wobei der Rest R-CO- ein zur Carbonsäure oxidierter Hydroxyethylstärkerest ist, der ein Molekulargewicht Mw von 5.000 bis 500.000 D aufweist.

5. Stärkederivate nach Anspruch 1 oder 2, wobei der Rest R-CO- ein zur Carbonsäure oxidierter Hydroxyethylstärkerest ist, der ein Molekulargewicht Mw von 8.000 bis 250.000 D aufweist.

6. Stärkederivate nach Anspruch 1-5, wobei der Rest R-CO- ein zur Carbonsäure oxidierter Hydroxyethylstärkerest ist, der einen Substitutionsgrad MS von 0,1 bis 0,8 aufweist.

7. Stärkederivate nach Anspruch 1-5, wobei der Rest R-CO- ein zur Carbonsäure oxidierter Hydroxyethylstärkerest ist, der einen Substitutionsgrad MS von 0,4 bis 0,7 aufweist.

8. Stärkederivate nach Anspruch 1-7, wobei der Rest R-CO- ein zur Carbonsäure oxidierter Hydroxyethylstärkerest ist, der ein Verhältnis von C₂:C₆ Substitution im Bereich von 2-12, bezogen auf die Hydroxyethylgruppen der Anhydroglucoseeinheiten, aufweist.

9. Stärkederivate nach Anspruch 1-7, wobei der Rest R-CO- ein zur Carbonsäure oxidierter Hydroxyethylstärkerest ist, der ein Verhältnis von C₂:C₆ Substitution im Bereich von 3-11, bezogen auf die Hydroxyethylgruppen der Anhydroglucoseeinheiten, aufweist.

10. Stärke-Wirkstoff-Konjugate der Formel (II) 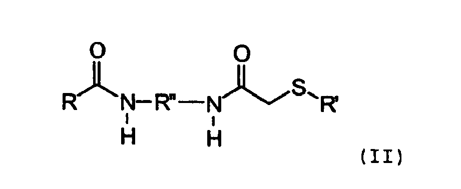 wobei R" eine geradkettige oder verzweigte Alkyl-, Aryl- oder Aralkylgruppe bedeutet, R-CO- einen oxidierten substituierten oder unsubstituierten Stärkerest bedeutet, der an der reduzierenden Endgruppe zu einer Carbonsäure oxidiert ist, und R' der Rest eines Wirkstoffs ist.

11. Stärke-Wirkstoff-Konjugate nach Anspruch 10, wobei R" eine Gruppe der Formel (CH₂)ₙ ist und n eine ganze Zahl von 1 bis 10 bedeutet.

12. Stärke-Wirkstoff-Konjugate nach Anspruch 10 oder 11, wobei der Rest R-CO- ein zur Carbonsäure oxidierter Hydroxyethylstärkerest ist, der ein Molekulargewicht Mw von 2.000 bis 1.000.000 D aufweist.

13. Stärke-Wirkstoff-Konjugate nach Anspruch 10 oder 11, wobei der Rest R-CO- ein zur Carbonsäure oxidierter Hydroxyethylstärkerest ist, der ein Molekulargewicht Mw von 5.000 bis 500.000 D aufweist.

14. Stärke-Wirkstoff-Konjugate nach Anspruch 10 oder 11, wobei der Rest R-CO- ein zur Carbonsäure oxidierter Hydroxyethylstärkerest ist, der ein Molekulargewicht Mw von 8.000 bis 250.000 D aufweist.

15. Stärke-Wirkstoff-Konjugate nach Anspruch 10-14, wobei der Rest R-CO- ein zur Carbonsäure oxidierter Hydroxyethylstärkerest ist, der einen Substitutionsgrad MS von 0,1 bis 0,8 aufweist.

16. Stärke-Wirkstoff-Konjugate nach Anspruch 10-14, wobei der Rest R-CO- ein zur Carbonsäure oxidierter Hydroxyethylstärkerest ist, der einen Substitutionsgrad MS von 0,4 bis 0,7 aufweist.

17. Stärke-Wirkstoff-Konjugate nach Anspruch 10-16, wobei der Rest R-CO- ein zur Carbonsäure oxidierter Hydroxyethylstärkerest ist, der ein Verhältnis von C₂:C₆ Substitution im Bereich von 2-12, bezogen auf die Hydroxyethylgruppen der Anhydroglucoseeinheiten, aufweist.

18. Stärke-Wirkstoff-Konjugate nach Anspruch 10-16, wobei der Rest R-CO- ein zur Carbonsäure oxidierter Hydroxyethylstärkerest ist, der ein Verhältnis von C₂:C₆ Substitution im Bereich von 3-11, bezogen auf die Hydroxyethylgruppen der Anhydroglucoseeinheiten, aufweist.

19. Stärke-Wirkstoff-Konjugate nach Anspruch 10-18, wobei der Wirkstoff ausgewählt ist aus einem Peptid, einem Protein, einem Antibiotikum, einer Nukleinsäure oder einem Hormon.

20. Stärke-Wirkstoff-Konjugate nach Anspruch 19, wobei es sich bei dem Protein um einen Antikörper, ein Antikörper-Fab-Fragment oder ein Antikörper-F(ab')₂-Fragment handelt.

21. Stärke-Wirkstoff-Konjugate nach Anspruch 19, wobei es sich bei dem Protein um Erythropoetin handelt.

22. Stärke-Wirkstoff-Konjugate nach Anspruch 19, wobei es sich bei dem Protein um ein Peptid oder Protein handelt, dem durch gezielte Mutagenese ein Cysteinrest eingefügt wurde.

23. Stärke-Wirkstoff-Konjugate nach Anspruch 19, wobei es sich um einen Wirkstoff handelt, dem durch Umsetzung mit 2-Iminothiolan eine SH-Funktion eingefügt wurde.

24. Stärke-Wirkstoff-Konjugate nach Anspruch 19, wobei es sich bei dem Wirkstoff um ein Cytokin handelt.

25. Stärke-Wirkstoff-Konjugate nach Anspruch 24, wobei das Cytokin ausgewählt ist aus Interferon α 2a und Interferon α 2b.

26. Verfahren zur Herstellung von Stärke-Wirkstoff-Konjugaten, **dadurch gekennzeichnet, dass** man
a) die reduzierenden Endgruppen einer substituierten oder unsubstituierten Stärke zur Carboxyl- oder Lactongruppe oxidiert,
b) die in Schritt a) hergestellte Carboxylgruppe bzw. das Lacton mit einem Diamin
H₂N-R"-NH₂
wobei R" eine geradkettige oder verzweigte Alkyl-, Aryl- oder Aralkylgruppe bedeutet, zu einer Verbindung der Formel (III) umsetzt 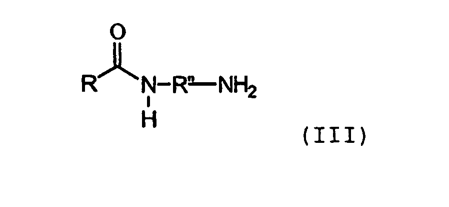 wobei R-CO- einen oxidierten substituierten oder unsubstituierten Stärkerest bedeutet, der an der reduzierenden Endgruppe zu einer Carbonsäure oxidiert ist,
c) die Verbindung der Formel (III) mit einer Halogenessigsäure und 1-Ethyl-3-(3-dimethylaminopropyl) carbodiimid als Aktivator zu einer Verbindung der Formel (I) umsetzt 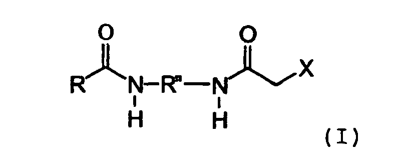 wobei X ein Brom oder Iodatom bedeutet, und
d) die Verbindung der Formel (I) mit einem mindestens eine SH-Gruppe enthaltenden Wirkstoff R'SH zu einem Stärke-Wirkstoff-Konjugat der allgemeinen Formel (II) umsetzt. 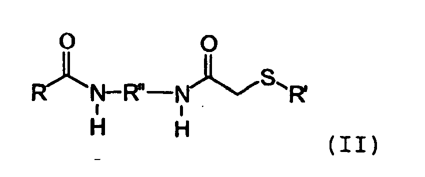 wobei R' einen Wirkstoffrest darstellt.

27. Verfahren nach Anspruch 26, **dadurch gekennzeichnet, dass** R" eine Gruppe der Formel (CH₂)ₙ ist, wobei n eine ganze Zahl von 1 bis 10 bedeutet.

28. Verfahren nach Anspruch 26 oder 27, **dadurch gekennzeichnet, dass** die Umsetzung des Schrittes d) bei einem pH-Wert zwischen 6,5 und 8,5 durchgeführt wird.

29. Arzneimittel, umfassend ein Stärke-Wirkstoff-Konjugat gemäß den Ansprüchen 10-25.

30. Verwendung von Stärke-Wirkstoff-Konjugaten gemäß den Ansprüchen 10-25 zur Herstellung eines Arzneimittels zur Behandlung von Infektionskrankheiten.

31. Verwendung von Stärke-Wirkstoff-Konjugaten gemäß den Ansprüchen 10-25 zur Herstellung eines Arzneimittels zur Behandlung hormoneller Störungen.

## Claims

1. Starch derivatives having the formula (I) 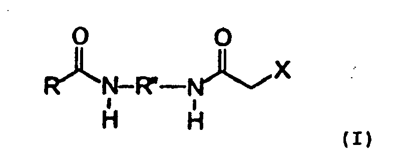 where X stands for a bromine- or iodine atom, R" a straight-chain or branched alkyl-, aryl- or aralkyl group and R-CO- an oxidised substituted or unsubstituted starch residue which is oxidised at the reducing end-group to a carboxylic acid.

2. Starch derivatives according to claim 1, where R" is a group having the formula (CH₂)ₙ and n is a whole number between 1 and 10.

3. Starch derivatives according to claim 1 or 2, where the residue R-CO- is a hydroxy ethyl starch residue with a weight average molecular weight Mw of 2,000 to1,000,000 D which is oxidised to carboxylic acid.

4. Starch derivatives according to claim 1 or 2, where the residue R-CO- is a hydroxy ethyl starch residue with a weight average molecular weight Mw of 5,000 to 500,000 D which is oxydised to carboxylic acid.

5. Starch derivatives according to claim 1 or 2, where the residue R-CO- is a hydroxy ethyl starch residue with a weight average molecular weight Mw of 8,000 to 250,000 D which is oxidised to carboxylic acid.

6. Starch derivatives according to claims 1-5, where the residue R-CO- is a hydroxy ethyl starch residue having a degree of substitution DS of 0.1 to 0.8 and which is oxidised to carboxylic acid.

7. Starch derivatives according to claims 1-5, where the residue R-CO- is a hydroxy ethyl starch residue having a degree of substitution DS of 0.4 to 0.7 and which is oxidised to carboxylic acid.

8. Starch derivatives according to claims 1-7, where the residue R-CO- is a hydroxy ethyl starch residue which exhibits a degree of C₂:C₆ substitution within the range of 2-12 with respect to the hydroxyethyl groups of the anhydroglucose units and which is oxidised to carboxylic acid.

9. Starch derivatives according to claims 1-7, where the R-CO- residue is a hydroxy ethyl starch residue which exhibits a degree of C₂:C₆ substitution within the range 3-11 with respect to the hydroxyethyl groups of the anhydroglucose units and which is oxidised to carboxylic acid.

10. Starch-active-agent-conjugates having the formula (II) 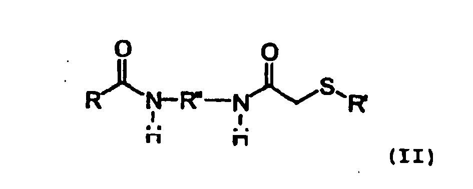 where R" stands for a straight-chain or a branched alkyl-, aryl or aralkyl group, R-CO- stands for an oxidised substituted or unsubstituted starch residue which is oxidised at the reducing end-group to a carboxylic acid and R' is the residue of an active agent.

11. Starch-active-agent-conjugates according to claim 10, where R" is a group having the formula (CH₂)ₙ and n stands for a whole number between and 10.

12. Starch-active-agent-conjugates according to claim 10 or 11, where the residue R-CO- is a hydroxy ethyl starch residue having a weight average molecular weight Mw of between 2,000 and 1,000,000 D and is oxidised to carboxylic acid.

13. Starch-active-agent-conjugates according to claim 10 or 11, where the residue R-CO- is a hydroxy ethyl starch residue having a weight average molecular weight Mw of between 5,000 and 500,000 D and is oxidised to carboxylic acid.

14. Starch-active-agent-conjugates according to claim 10 or 11, where the residue R-CO- is a hydroxy ethyl starch residue having a weight average molecular weight Mw of between 8,000 and 250,000 D and is oxidised to carboxylic acid.

15. Starch-active-agent conjugates according to claims 10-14, where the residue R-CO- is a hydroxy ethyl starch residue having a degree of substitution DS of between 0.1 and 0.8 and is oxidised to carboxylic acid.

16. Starch-active-agent-conjugates according to claims 10-14, where the residue R-CO- is a hydroxy ethyl starch residue having a degree of substitution DS of between 0.4 and 0.7 and is oxidised to carboxylic acid.

17. Starch-active-agent-conjugates according to claims 10-14, where the residue R-CO- is a hydroxy ethyl starch residue having a degree of C₂:C₆ substitution in the range between 2 and 12 with respect to hydroxyethyl groups of the anhydrous glucose units and is oxidised to carboxylic acid.

18. Starch-active-agent-conjugates according to claims 10-16, where the R-CO- residue is a hydroxy ethyl starch residue having a degree of C₂:C₆- substitution in the range between 3 and 11 with respect to the hydroxyethyl groups of the anhydrous glucose units and is oxidised to carboxylic acid.

19. Starch-active-agent-conjugates according to claims 10-18, where the selected active agent consists of a peptide, a protein, an antibiotic, a nucleic acid or a hormone.

20. Starch-active-agent-conjugates according to claim 19, where the protein is an anti-body, an anti-body-Fab-fragment or an anti-body F(ab')₂-fragment.

21. Starch-active-agent-conjugates according to claim 19, where the protein is erythropoietin.

22. Starch-active-agent-conjugates according to claim 19, where the protein is a peptide or a protein into which a cysteine residue has been introduced by targeted mutagenesis.

23. Starch-active-agent-conjugates according to claim 19, where an active agent is involved into which an SH-function has been introduced by reaction with 2-iminothiolane.

24. Starch-active-agent-conjugates according to claim 19 where the active agent is a cytokine.

25. Starch-active-agent-conjugates according to claim 24, where the selected cytokine consists of interferon α 2a and interferon α 2b.

26. Process for the manufacture of starch-active agent-conjugates, **characterised in that**
a) the reducing end-groups of a substituted or unsubstituted starch is oxidised to the carboxyl- or lactone group,
b) the carboxyl-group or the lactone produced in step a) is reacted with a diamine
H₂N-R"-NH₂
where R" stands for a straight-chain- or branched alkyl, aryl- or aralkyl group to result in a compound having the formula (III), 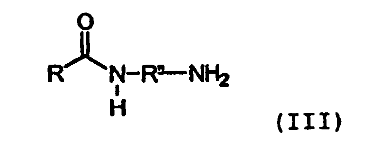 where R-CO- stands for an oxidised substituted or unsubstituted starch residue which is oxidised at the reducing end to a carboxylic acid,
c) the compound having the formula (III) is reacted with a halogenated acetic acid and 1-ethyl-3-(3-dimethylaminopropyl) carbodiimide as activator to produce a compound having the formula (I) 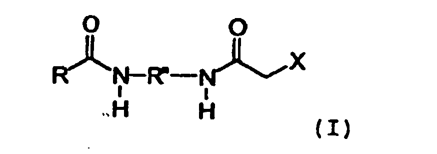 where X stands for a bromine- or an iodine atom, and
d) the compound having the formula (I) is reacted with an active agent R'SH having at least one SH-containing group to form a starch-active-agent-conjugate having the general formula (II) 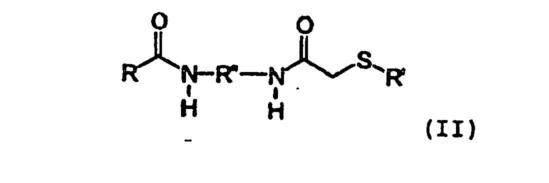 where R' represents an active agent residue.

27. Process according to claim 26, **characterised in that** R" is a group having the formula (CH₂)ₙ, where n is a whole number between 1 and 10.

28. Process according to claim 26 or 27, **characterised in that** the reaction of step d) is carried out at a pH-value of between 6.5 and 8.5.

29. Medical drug comprising a starch-active-agent conjugate according to claims 10 - 25.

30. Use of the starch-active-agent-conjugates according to claims 10 - 25 for the manufacture of a medical drug for the treatment of infective illnesses.

31. Use of starch-active-agent-conjugates according to claims 10 - 25 for the manufacture of a medical drug for the treatment of hormonal disturbances.

## Revendications

1. Dérivés d'amidon de formule (I) 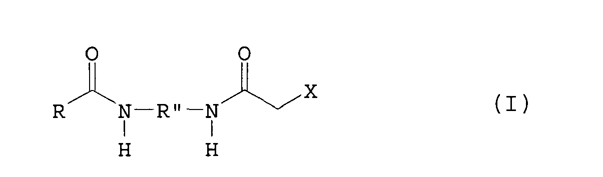 dans laquelle X désigne un atome de brome ou d'iode, R" désigne un groupe alkyle, aryle ou aralkyle à chaîne droite ou ramifiée et R-CO- désigne un résidu amidon oxydé, substitué ou non substitué, qui est oxydé en un acide carboxylique au niveau du groupe terminal réducteur.

2. Dérivés d'amidon selon la revendication 1, dans lesquels R" est un groupe de formule (CH₂)ₙ et n est un nombre entier valant de 1 à 10.

3. Dérivés d'amidon selon la revendication 1 ou la revendication 2, dans lesquels le résidu R-CO- est un résidu hydroxyéthylamidon oxydé en acide carboxylique qui présente une masse moléculaire Mw de 2 000 à 1 000 000 daltons.

4. Dérivés d'amidon selon la revendication 1 ou la revendication 2, dans lesquels le résidu R-CO- est un résidu hydroxyéthylamidon oxydé en acide carboxylique qui présente une masse moléculaire Mw de 5 000 à 500 000 daltons.

5. Dérivés d'amidon selon la revendication 1 ou la revendication 2, dans lesquels le résidu R-CO- est un résidu hydroxyéthylamidon oxydé en acide carboxylique qui présente une masse moléculaire Mw de 8 000 à 250 000 daltons.

6. Dérivés d'amidon selon les revendications 1 à 5, dans lesquels le résidu R-CO- est un résidu hydroxyéthylamidon oxydé en acide carboxylique qui présente un degré de substitution MS de 0,1 à 0,8.

7. Dérivés d'amidon selon les revendications 1 à 5, dans lesquels le résidu R-CO- est un résidu hydroxyéthylamidon oxydé en acide carboxylique qui présente un degré de substitution MS de 0,4 à 0,7.

8. Dérivés d'amidon selon les revendications 1 à 7, dans lesquels le résidu R-CO- est un résidu hydroxyéthylamidon oxydé en acide carboxylique qui présente un ratio de substitution C₂:C₆ dans la plage de 2 à 12, rapporté aux groupes hydroxyéthyle des motifs anhydroglucose.

9. Dérivés d'amidon selon les revendications 1 à 7, dans lesquels le résidu R-CO- est un résidu hydroxyéthylamidon oxydé en acide carboxylique qui présente un ratio de substitution C₂ : C₆ dans la plage de 3 à 11, rapporté aux groupes hydroxyéthyle des motifs anhydroglucose.

10. Conjugués amidon/principe actif de formule (II) 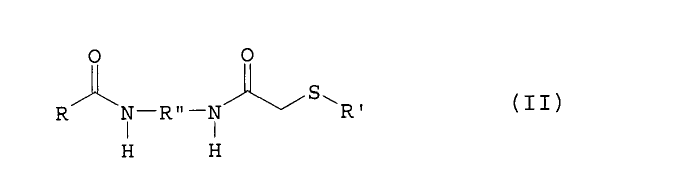 dans laquelle R" désigne un groupe alkyle, aryle ou aralkyle à chaîne droite ou ramifiée, R-CO- désigne un résidu amidon oxydé, substitué ou non substitué, qui est oxydé en un acide carboxylique au niveau du groupe terminal réducteur, et R' est le résidu d'un principe actif.

11. Conjugués amidon/principe actif selon la revendication 10, dans lesquels R" est un groupe de formule (CH₂)ₙ et n est un nombre entier valant de 1 à 10.

12. Conjugués amidon/principe actif selon la revendication 10 ou la revendication 11, dans lesquels le résidu R-CO- est un résidu hydroxyéthylamidon oxydé en acide carboxylique qui présente une masse moléculaire Mw allant de 2 000 à 1 000 000 daltons.

13. Conjugués amidon/principe actif selon la revendication 10 ou la revendication 11, dans lesquels le résidu R-CO- est un résidu hydroxyéthylamidon oxydé en acide carboxylique qui présente une masse moléculaire Mw allant de 5 000 à 500 000 daltons.

14. Conjugués amidon/principe actif selon la revendication 10 ou la revendication 11, dans lesquels le résidu R-CO- est un résidu hydroxyéthylamidon oxydé en acide carboxylique qui présente une masse moléculaire Mw allant de 8 000 à 250 000 daltons.

15. Conjugués amidon/principe actif selon les revendications 10 à 14, dans lesquels le résidu R-CO- est un résidu hydroxyéthylamidon oxydé en acide carboxylique qui présente un degré de substitution MS de 0,1 à 0,8.

16. Conjugués amidon/principe actif selon les revendications 10 à 14, dans lesquels le résidu R-CO- est un résidu hydroxyéthylamidon oxydé en acide carboxylique qui présente un degré de substitution MS de 0,4 à 0,7.

17. Conjugués amidon/principe actif selon les revendications 10 à 16, dans lesquels le résidu R-CO- est un résidu hydroxyéthylamidon oxydé en acide carboxylique qui présente un ratio de substitution C₂:C₆ dans la plage de 2 à 12, rapporté aux groupes hydroxyéthyle des motifs anhydroglucose.

18. Conjugués amidon/principe actif selon revendications 10 à 16, dans lesquels le résidu R-CO- est un résidu hydroxyéthylamidon oxydé en acide carboxylique qui présente un ratio de substitution C₂:C₆ dans la plage de 3 à 11, rapporté aux groupes hydroxyéthyle des motifs anhydroglucose.

19. Conjugués amidon/principe actif selon les revendications 10 à 18, dans lesquels le principe actif est choisi parmi un peptide, une protéine, un antibiotique, un acide nucléique ou une hormone.

20. Conjugués amidon/principe actif selon la revendication 19, dans lesquels la protéine est un anticorps, un fragment d'anticorps Fab ou un fragment d'anticorps F(ab')₂.

21. Conjugués amidon/principe actif selon la revendication 19, dans lesquels la protéine est l'érythropoïétine.

22. Conjugués amidon/principe actif selon la revendication 19, dans lesquels la protéine est un peptide ou une protéine à laquelle un résidu cystéine a été ajouté par mutagenèse dirigée.

23. Conjugués amidon/principe actif selon la revendication 19, dans lesquels le principe actif est un principe actif auquel une fonction SH a été ajoutée par réaction avec du 2-iminothiolane.

24. Conjugués amidon/principe actif selon la revendication 19, dans lesquels le principe actif est une cytokine.

25. Conjugués amidon/principe actif selon la revendication 24, dans lesquels la cytokine est choisie parmi l'interféron α 2a et l'interféron α 2b.

26. Procédé de production de conjugués amidon/principe actif, **caractérisé en ce que**
a) on oxyde les groupes terminaux réducteurs d'un amidon substitué ou non substitué en un groupe carboxyle ou lactone,
b) on fait réagir le groupe carboxyle ou la lactone obtenu(e) à l'étape a) avec une diamine
H₂N-R"-NH₂
où R" désigne un groupe alkyle, aryle ou aralkyle à chaîne droite ou ramifiée, pour donner un composé de formule (III) 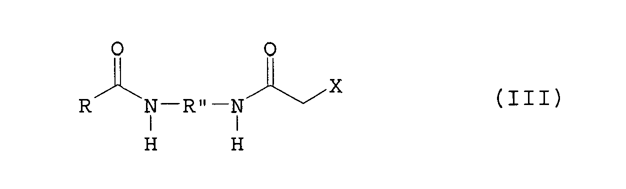 dans laquelle R-CO- désigne un résidu amidon oxydé, substitué ou non substitué, qui est oxydé en un acide carboxylique au niveau du groupe terminal réducteur,
c) on fait réagir le composé de formule (III) avec un acide haloacétique et du 1-éthyl-3-(3-diméthylaminopropyl)carbodiimide comme activateur pour donner un composé de formule (I) 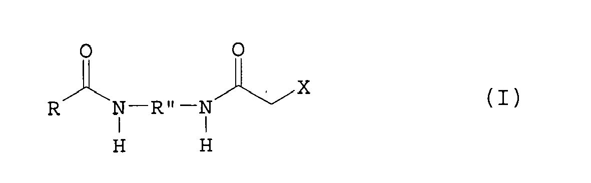 où X désigne un atome de brome ou d'iode, et
d) on fait réagir le composé de formule (I) avec un principe actif R'SH contenant au moins un groupe SH pour donner un conjugué amidon/principe actif de la formule générale (II) 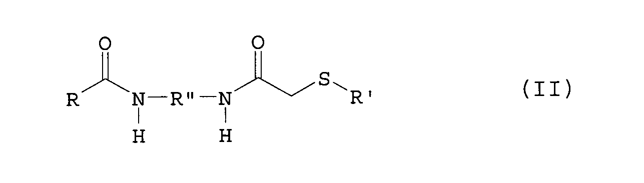 où R' représente un résidu de principe actif.

27. Procédé selon la revendication 26, **caractérisé en ce que** R" est un groupe de formule (CH₂)ₙ, où n est un nombre entier valant de 1 à 10.

28. Procédé selon la revendication 26 ou la revendication 27, **caractérisé en ce que** la réaction de l'étape d) est effectuée à un pH compris entre 6,5 et 8,5.

29. Médicament comprenant un conjugué amidon/principe actif selon les revendications 10 à 25.

30. Utilisation de conjugués amidon/principe actif selon les revendications 10 à 25 pour préparer un médicament destiné au traitement de maladies infectieuses.

31. Utilisation de conjugués amidon/principe actif selon les revendications 10 à 25 pour préparer un médicament destiné au traitement de troubles hormonaux.
